(19) Europäisches Patentamt
European Patent Office
Office européen des brevets

(11) Numéro de publication: **0 292 363 B1**

(12) **FASCICULE DE BREVET EUROPEEN**

(45) Date de publication de fascicule du brevet: **21.04.93**

(51) Int. Cl.$^5$: **C01B 33/20**, C07C 1/24

(21) Numéro de dépôt: **88401147.9**

(22) Date de dépôt: **11.05.88**

(54) **Zéolites de structures MFI à base de silice et d'oxyde de titane et procédé de synthèse de celles-ci.**

(30) Priorité: **22.05.87 FR 8707187**
**30.03.88 FR 8804167**

(43) Date de publication de la demande:
**23.11.88 Bulletin 88/47**

(45) Mention de la délivrance du brevet:
**21.04.93 Bulletin 93/16**

(84) Etats contractants désignés:
**AT BE CH DE ES FR GB GR IT LI LU NL SE**

(56) Documents cités:
**EP-A- 0 132 550**
**WO-A-85/04853**
**FR-A- 2 429 182**
**FR-A- 2 471 950**

**CHEMICAL ABSTRACTS, vol. 104, no. 2, 13 janvier 1986, page 117, résumé no. 7808u, Columbus, Ohio, US; & JP-A-60 127 217 (JAPAN SYNTHETIC RUBBER CO., LTD) 06-07-1985**

(73) Titulaire: **RHONE-POULENC CHIMIE**
**25, quai Paul Doumer**
**F-92408 Courbevoie Cédex(FR)**

(72) Inventeur: **Popa, Jean-Michel**
**2, rue Roger Breton**
**F-93700 Drancy(FR)**
Inventeur: **Guth, Jean-Luc**
**59, rue Belle Vue Brunstatt**
**F-68200 Mulhouse(FR)**
Inventeur: **Kessler, Henri**
**17, rue de la Forêt**
**F-68270 Wittenheim(FR)**

(74) Mandataire: **Dutruc-Rosset, Marie-Claude et al**
**RHONE-POULENC CHIMIE Direction des Brevets 25, Ouai Paul Doumer**
**F-92408 Courbevoie Cédex (FR)**

**EP 0 292 363 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne des zéolites à base de silice et d'oxyde de titane.

Elle concerne plus particulièrement des zéolites de structure MFI, et un procédé de synthèse de celles-ci.

Les zéolites sont des tectosilicates cristallisés. Les structures sont constituées par des assemblages de tétraèdres $TO_4$ formant une charpente tridimensionnelle par la mise en commun des oxygènes. Dans les zéolites du type aluminosilicate qui sont les plus communes, T représente le silicium tétravalent ainsi que l'aluminium trivalent. Les cavités et canaux de dimensions moléculaires de cette charpente accueillent les cations compensant le déficit de charge lié à la présence de l'aluminium trivalent dans les tétraèdres. On connaît aussi quelques rares zéolites où le silicium est remplacé par du germanium tétravalent. De même des éléments trivalents comme le gallium et plus rarement le bore ou le beryllium peuvent se substituer à l'aluminium.

D'une manière générale, la composition des zéolites peut être représentée par la formule brute $M_{2/n}O$ ; $Y_2O_3$ ; $xZO_2$ à l'état déshydraté et calciné. Z et Y représentent respectivement les éléments tétravalent et trivalent des tétraèdres $TO_4$ ; M représente un élément électropositif de valence n tel qu'un alcalin ou alcalino-terreux et constituant les cations de compensation ; x peut varier de 2 à théoriquement l'infini auquel cas la zéolite est une silice cristallisée.

Chaque type de zéolite possède une structure poreuse distincte. La variation des dimensions et formes des pores d'un type à l'autre, entraîne des changements des propriétés adsorbantes. Seules les molécules ayant certaines dimensions et formes sont capables d'entrer dans les pores d'une zéolite particulière. En raison de ces caractéristiques remarquables les zéolites conviennent tout particulièrement pour la purification ou la séparation de mélanges gazeux ou liquides comme par exemple la séparation d'hydrocarbures par adsorption sélective.

La composition chimique avec en particulier la nature des éléments présents dans les tétraèdres $TO_4$ et la nature des cations de compensation échangeables, est également un facteur important intervenant dans la sélectivité de l'adsorption et surtout dans les propriétés catalytiques de ces produits. Ils sont utilisés comme catalyseurs ou supports de catalyseurs dans le craquage, le reformage et la modification d'hydrocarbures ainsi que dans l'élaboration de nombreuses molécules.

De nombreuses zéolites existent dans la nature : ce sont des aluminosilicates dont les disponibilités et propriétés ne répondent pas toujours aux exigences des applications industrielles. De ce fait, la recherche de produits ayant des propriétés nouvelles a conduit à la synthèse d'une grande variété de zéolites essentiellement du type aluminosilicate. Parmi les nombreux exemples de ce type on peut signaler : la zéolite A (Brevet US N° 2882243), la zéolite X (Brevet US N° 2882244), la zéolite Y (Brevet US N° 3130007), la zéolite L (Brevet français N° 1224154), la zéolite T (Brevet français N° 1223775), la zéolite ZSM5 (Brevet US N° 3702886), la zéolite ZSM12 (Brevet US N° 3832444), la zéolite ZSM48 (Brevet européen N° 0015132).

Il a également été proposé des zéolites contenant du titane dans les tétraèdres $TO_4$. On peut citer à titre d'illustration, les brevets français N° 2 471 950, européens N° 104 107, 100 119 et américain N° 3 329 481. Toutefois, la liaison du titane dans un système cristallin est plutôt du type octaédrique que tétraédrique, la substitution du silicium par le titane dans la structure d'une zéolite est très difficile à obtenir dans les aluminosilicates.

Les zéolites sont généralement obtenues à partir d'un mélange réactionnel qui se transforme, en milieu hydrothermal, par un procédé de dissolution-recristallisation, le précipité cristallin étant, après séparation et séchage, calciné pour donner une zéolite active.

Le mélange réactionnel contient des réactifs pouvant fournir les éléments T à incorporer dans la charpente de la zéolite, ces réactifs sont généralement des gels aqueux contenant des oxydes ou hydroxydes des éléments T.

Il contient également un mobilisateur favorisant la dissolution de ces réactifs et le transfert depuis la phase aqueuse sur les cristaux de zéolites en formation, et un agent structurant permettant, par son incorporation, la formation d'espaces microporeux ainsi qu'une stabilisation de la zéolite.

Comme mobilisateur, les ions hydroxydes sont utilisés. Ainsi, les milieux réactionnels ont généralement un pH supérieur à 10, pour d'une part assurer la dissolution des sources de silice et des autres sources des éléments T, et d'autre part, faciliter le transfert des espèces solubles sur la zéolite en voie de formation.

Les zéolites contenant des espèces facilement solubles en milieu basique, telles que, par exemple, l'aluminium, sont très bien synthétisées par cette méthode.

Toutefois, il apparaît très difficile d'incorporer du titane dans les tétraèdres $TO_4$ de la charpente de la zéolite quand le milieu réactionnel est basique.

Par ailleurs, en milieu basique, les zéolites métastables ne peuvent être obtenues que si le milieu réactionnel est sursaturé en espèces actives. Ceci entraîne une nucléation rapide conduisant à des cristaux de zéolite de petites dimensions, sans possibilité de contrôle aisé de la dimension de ces cristaux.

De plus, les procédés de synthèse utilisant des mélanges réactionnels basiques nécessitent l'emploi comme cation de compensation, des cations alcalins ou alcalino-terreux. Ces cations doivent souvent être éliminés par la suite car ils affectent les propriétés catalytiques ou adsorbantes de la zéolite. Cette élimination est généralement réalisée par des échanges répétés d'ions avec des cations $NH_4^+$. La zéolite contenant les cations ammonium est ensuite calcinée pour éliminer ceux-ci sous forme de $NH_3$.

L'invention a notamment pour but de remédier à ces inconvénients en proposant une zéolite de la famille des pentasils et s'apparentant aux zéolites du type MFI à base de silice et d'oxyde de titane, et un procédé de synthèse de cette zéolite à partir de mélanges réactionnels neutres ou acides permettant notamment d'obtenir une incorporation du titane dans la charpente de la zéolite dans de grande proportion ainsi que des cristaux de zéolite de dimensions totalement contrôlées. Cette zéolite pourra être appelée ci-après titanozéosilite.

A cet effet, l'invention a pour objet une zéolite de structure MFI à base de silice et d'oxyde de titane et ayant, après calcination, la formule suivante :

$$\left[ Si_{(96-x)}, Ti_x \right] O_{192}$$

dans laquelle :

x est compris entre 0,1 et 6 environ.

La zéolite de l'invention a un système cristallin monoclinique et un diagramme de diffraction des rayons X défini dans le tableau I.

Dans ce tableau, les valeurs extrêmes des différentes équi-distances réticulaires $d_{hkl}$ sont données et correspondent aux concentrations limites de titane incorporé dans la charpente de la zéolite, ou plus précisément au rapport Ti/Si.

En effet, l'identification des zéolites de l'invention à structure MFI peut être notamment et avantageusement réalisée par l'établissement de leur diagramme de diffraction des rayons X.

Ce diagramme de diffraction peut être obtenu à l'aide d'un diffractomètre et utilisant la méthode classique des poudres avec le rayonnement $K\alpha$ du cuivre. A partir de la position des pics de diffraction représentée par l'angle $2\theta$ on calcule, par la relation de Bragg, les équi-distances réticulaires $d_{hkl}$ caractéristiques de l'échantillon. L'estimation de l'erreur de mesure $\Delta(d_{hkl})$ sur $d_{hkl}$ se calcule, en fonction de l'erreur absolue $\Delta(2\theta)$ affectée à la mesure de $2\theta$, par la relation de Bragg. Une erreur absolue $\Delta$ $(2\theta)$ égale à $\pm$ 0,2° est couramment admise. L'intensité relative $I/I_o$ affectée à chaque valeur de $d_{hkl}$ est estimée à partir de la hauteur du pic de diffraction correspondant. On utilise souvent une échelle de symboles pour caractériser cette intensité : FF = très fort, F = fort, mF = moyen à fort, m = moyen, mf = moyen à faible, f = faible, ff = très faible.

TABLEAU I

diagramme de diffraction des rayons X

| Valeurs extrêmes des $d_{hkl}$ (nm) | $I/I_o$ | Valeurs extrêmes des $d_{hkl}$ (nm) | $I/I_o$ |
|---|---|---|---|
| 1,110-1,128 | F-FF | 0,3785-0,3845 | mF |
| 0,991-1,012 | F-FF | 0,3735-0,3795 | m |
| 0,972-0,986 | f | 0,3715-0,3775 | m |
| 0,895-0,906 | ff | 0,3705-0,3765 | m |
| 0,803-0,813 | ff | 0,3645-0,3700 | f |
| 0,741-0,753 | ff (large) | 0,3610-0,3670 | f |
| 0,704-0,715 | ff (large) | 0,3470-0,3525 | ff |
| 0,666-0,678 | f | 0,3430-0,3485 | f(f) |
| 0,632-0,643 | f | 0,3415-0,3470 | f(f) |
| 0,595-0,605 | mf | 0,3385-0,3439 | ff |
| 0,589-0,598 | f | 0,3341-0,3394 | f(f) |
| 0,568-0,577 | mf | 0,3290-0,3345 | f (large) |
| 0,565-0,574 | f (épaulement) | 0,3240-0,3292 | f |
| 0,555-0,564 | f | 0,3045-0,3099 | f(f) |
| 0,534-0,543 | f(f) | 0,3020-0,3068 | f |
| 0,531-0,539 | f(f) | 0,2978-0,3026 | f |
| 0,510-0,518 | ff | 0,2952-0,2999 | ff (épaulement) |
| 0,502-0,508 | ff | 0,2944-0,2991 | f |
| 0,496-0,504 | mf | 0,2914-0,2961 | ff |
| 0,485-0,493 | ff | 0,2852-0,2898 | ff (large) |
| 0,468-0,476 | ff | 0,2774-0,2818 | ff |
| 0,459-0,466 | f | 0,2722-0,2766 | ff (large) |
| 0,444-0,451 | f | 0,2676-0,2720 | ff |
| 0,433-0,441 | f | 0,2606-0,2648 | ff |
| 0,423-0,431 | f | 0,2586-0,2627 | ff |
| 0,4055-0,4125 | ff | 0,2544-0,2585 | ff (large) |
| 0,3985-0,4045 | f | 0,2508-0,2548 | ff |
| 0,3835-0,3905 | F | 0,2478-0,2518 | f |
| 0,3805-0,3865 | mF | | |

Selon une autre caractéristique de l'invention, la zéolite contient du fluor, la concentration en fluor est avantageusement comprise entre 0,01 et 0,8 % en poids après calcination.

Toutefois, le fluor peut être éliminé sans pour cela modifier la structure de la zéolite conforme à l'invention.

L'invention a également pour objet un procédé de synthèse d'une zéolite conforme à l'invention, ce procédé comprend :

(i) la préparation d'un mélange réactionnel en milieu aqueux contenant au moins une source d'oxyde de silicium, une source d'oxyde de titane, des ions fluorures, et un agent structurant, le pH de ce mélange réactionnel étant compris entre 1,5 environ et 10,5 environ,

(ii) la cristallisation de ce mélange réactionnel et la récupération du précipité cristallin, et

(iii) la calcination de celui-ci à une température supérieure à 450 °C.

Le diagramme de diffraction des rayons X donné dans le tableau I est celui d'une zéolite soumise à une calcination comme indiqué ci-dessus.

L'emploi d'ions fluorures dans le milieu réactionnel, qui jouent le rôle d'agent mobilisateur, permet d'obtenir une solubilisation des espèces T (Si et Ti) dans un milieu ayant un pH inférieur à 10. Ainsi, il est possible d'utiliser comme cations de compensation des ions $NH_4^+$, qui pourront être éliminés totalement, si on le désire, lors de la calcination.

De plus, la cristallisation s'effectuant dans un milieu à pH inférieur à 10, la vitesse de nucléation est plus lente. Ainsi, il est possible d'obtenir des cristaux de zéolites contrôlés par pilotage de la vitesse de nucléation.

Les rapports molaires entre les différentes espèces dans le milieu réactionnel sont compris pour Ti/Si entre 1,5 environ et 0,002 environ, F/Si entre 10 et 0,04 environ, $H_2O$/Si entre 400 environ et 4 environ et pour l'agent structurant par rapport à l'espèce silicium entre 2 environ et 0,02 environ.

Avantageusement, le rapport molaire Ti/Si est compris entre 1 et 0,01, F/Si entre 6 et 0,06, $H_2O$/Si entre 100 et 6 et entre l'agent structurant et l'espèce silicium entre 1 et 0,04.

De nombreuses sources de silice peuvent être utilisées. On peut citer, à titre d'exemple, les silices sous formes d'hydrogels, d'aérogels, de suspensions colloïdales, les silices résultant de la précipitation à partir de solutions de silicates solubles ou de l'hydrolyse d'esters siliciques comme $Si(OC_2H_5)_4$ ou de complexes comme $Na_2SiF_6$, les silices préparées par des traitements d'extraction et d'activation de composés cristallisés naturels ou synthétiques comme les silicates d'aluminium, les aluminosilicates, les argiles. On peut également utiliser des composés du silicium tétravalent hydrolysables tels que les halogénures de silicium.

Parmi les sources d'oxyde de titane, on peut citer, à titre d'exemple, les oxydes et les hydroxydes de titane, cristallisés ou amorphes, les composés du titane tétravalent pouvant être hydrolysés tels les halogénures ($TiCl_4$), les alcoolates, les sels solubles de titane tels $TiOSO_4$, $(NH_4)_2$ $TiO(C_2O_4)_2$.

Il est également possible d'utiliser comme sources de silice ou d'oxyde de titane, des composés comprenant les éléments Si et Ti tels que, par exemple, des verres ou gels à base des oxydes de ces deux éléments.

Les sources de silice et d'oxyde de titane peuvent être engagées sous forme soluble ou de solides pulvérulents mais également sous forme d'aggomérats tels que, par exemple, pastilles, extrudés pouvant être transformés en zéolite de structure désirée sans modification de forme.

Les anions fluorures peuvent être introduits sous forme d'acide fluorhydrique, de sels tels que, par exemple, $NH_4F$, $NH_4HF_2$, $NH(C_3H_7)_3F$, $N(C_3H_7)_4F$, de composés hydrolysables libérant les anions fluorures dans le milieu réactionnel, tels que, par exemple $SiF_4$, $(NH_4)_2SiF_6$, $(NH_4)_2Ti\ F_6$ ou analogues.

Le fluorure d'ammonium ou le fluorure d'ammonium acide sont des sels préférés. En effet ces sels sont très solubles et n'apportent aucun élément indésirable et, de plus, ils sont facilement éliminables en fin de cristallisation.

Les agents structurants convenables pour l'invention sont :

- les amines de formule I :

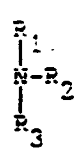

dans laquelle :

$R_1$, $R_2$, $R_3$ identiques ou différents représentent un groupe alkyle, de préférence un groupe propyle ou butyle.

- les ammonium quaternaires de formule II

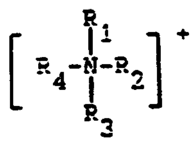

dans laquelle :

$R_1$, $R_2$, $R_3$, $R_4$ identiques ou différents représentent des groupes alkyles, de préférence des groupes propyles ou butyles.

- les composés de formules I et II dans lesquelles l'azote a été remplacé par un atome de phosphore.

Selon une caractéristique préférée de l'invention, les agents structurants sont la tripropylamine ou les composés qui peuvent fournir des cations tétrapropylammonium.

Avantageusement, l'agent structurant est ajouté au mélange réactionnel sous forme d'un sel d'une amine ou d'un ammonium quaternaire fournissant les cations ci-dessus mentionnés.

Selon une autre caractéristique de l'invention, le mélange réactionnel peut comprendre un agent co-mobilisateur du titane tétravalent dans un rapport molaire par rapport au silicium comprise entre 3 et 0,01 et avantageusement entre 2 et 0,04.

Les agents co-mobilisateurs convenables pour l'invention sont, par exemple, l'acide oxalique et ses sels, l'acétylacétone, l'acide tartrique et ses sels.

La cristallisation de la zéolite peut être obtenue par chauffage du mélange réactionnel à une température comprise entre 50 ° C environ at 240 ° C environ, de préférence entre 75 ° C et 225 ° C pendant le temps nécessaire à la cristallisation, selon un mode opératoire classique de synthèse de zéolite et connu de l'homme du métier. A titre indicatif, la durée de chauffage peut être comprise entre 6 heures et 500 heures environ.

Ce chauffage et cette cristallisation sont réalisés de préférence dans un récipient ou autoclave revêtu d'une couche telle que, par exemple, le polytétrafluoroéthane.

Le mélange réactionnel peut être agité ou non.

Après cristallisation, le précipité obtenu est recueilli, par exemple, par filtration.

Ce précipité est ensuite chauffé après un séchage éventuel, à une température supérieure à 450 ° C, de préférence supérieure à 500 ° C, afin de décomposer par calcination ou décomposition thermique les espèces organiques contenues dans le précipité, telles que, par exemple, l'agent structurant, les cations de compensation ($NH_4^+$).

Les titanozéosilites de l'invention sont des adsorbants sélectifs.

Ces composés ont également comme caractéristique importante de posséder des propriétés catalytiques qui permettent leur utilisation comme catalyseur ou support de catalyseur pour des réactions de transformation de composés organiques variés telles que par exemple:

L'alkylation d'hydrocarbures comme le benzène et le toluène, l'isomérisation des paraffines et naphtènes, la conversion d'éthers ou d'alcools en hydrocarbures, l'oxydation, la dismutation de composés aromatiques comme le toluène, le reformage, le craquage et l'hydrocraquage, la polymérisation des composés à liaison acétyléniques, l'hydrogénation et deshydrogénation des hydrocarbures, la deshydratation des composés aliphatiques, la conversion de composés carbonyles aliphatiques ou d'oléfines, la méthanation.

L'invention a également pour objet un produit cristallin du type zéolite à structure MFI à base de silice et d'oxyde de titane susceptible d'être obtenu par le procédé comprenant :

(i) la préparation d'un mélange réactionnel en milieu aqueux contenant au moins une source de silice, une source d'oxyde de titane, des ions fluorures et un agent structurant, le pH du mélange réactionnel étant compris entre environ 1,5 et 10,5.

(ii) la cristallisation du mélange réactionnel et la récupération du précipité cristallin.

Les rapports molaires des différentes espèces dans le milieu réactionnel sont ceux indiqués précédemment.

Le précipité cristallin est avantageusement lavé pour éliminer les impuretés et notamment les cations ou anions non accrochés ou incorporés dans la structure.

Ce produit manipulable est notamment et principalement utilisé pour la production de zéolite du type MFI par calcination sous des conditions appropriées et déterminées en fonction de l'utilisation désirée de la zéolite.

D'autres buts, caractéristiques et détails de l'invention apparaîtront plus clairement au vu des exemples suivants donnés uniquement à titre indicatif et illustratif.

Exemple 1

On dissout 13,8 g de $TiO_2$ type anatase dans 51,8 g d'une solution aqueuse contenant 50 % HF. On ajoute à cette solution 12,5 g de silice Aerosil ($SiO_2$). Après malaxage, on ajoute une deuxième solution obtenue par dissolution de 27,8 g de bromure de tétrapropylammonium (TPABr) dans 44,5 g d'eau et on agite. Puis on ajoute, toujours sous agitation une solution aqueuse à 25 % d'ammoniac jusqu'à ce que le pH soit compris entre 6 et 6,5. On obtient ainsi un gel homogène auquel on ajoute 0,25 g d'une zéolite à structure MFI comme germes de cristallisation.

Les rapports molaires dans le mélange réactionnel sont :
Ti/Si = 0,83 ; F/Si = 6,2 ; $TPA^+$/Si = 0,5 ; $NH_4^+$/Si = 3,3 ; $H_2O$/Si = 28.

Le mélange réactionnel est transféré dans un autoclave gainé par du polytétrafluoroéthane et chauffé pendant 2 jours à 170 ° C.

Après filtration, lavage à l'eau chaude et séchage, on obtient 8,5 g de zéolite type MFI pure, ou titanozéosilite identifiée par le diagramme de diffraction des rayons X établi sur le produit calciné. Les valeurs mesurées pour $d_{hkl}$ et $I/I_o$ sont conformes aux valeurs du tableau I.

La taille des cristaux est comprise entre 0,5 et 5 $\mu$m. Après calcination à 550 ° C pendant 4 h, l'analyse chimique du produit obtenu donne un rapport Si/Ti de 26, une teneur de F de 0,14 %.

Exemple 2

On verse lentement sous forte agitation 12,5 g de $Si(OC_2H_5)_4$ puis 5,10 g de $Ti(OC_4H_9)_4$ dans une solution contenant 32,4 g d'eau, 4 g de TPA-Br et 1,1 g de $NH_4F$. On ajoute 0,072 g de cristaux de structure MFI comme germe. Le pH est de 7,5.

La composition molaire du gel ainsi obtenu est caractérisée par les rapports molaires suivants :
Ti/Si = 0,25 ; F/Si = 0,5 ; $TPA^+$/Si = 0,25 ; $NH_4^+$/Si = 0,5 ; $H_2O$/Si = 30.

Ce gel est chauffé dans un autoclave revêtu de polytétrafluoroéthane (PTFE) pendant 6 jours à 200 ° C. Le pH du milieu, à l'ouverture de l'autoclave est de 8,5. Après séparation et lavage à l'eau chaude, on obtient 4,9 g de solide dont l'analyse radiocristallographique révèle qu'il s'agit essentiellement de zéolite du type MFI avec une impureté cristallisé du type anatase. L'examen au microscope polarisant montre également la présence de matière amorphe.

Exemple 3

On prépare un gel mixte contenant les éléments Si et Ti en réalisant un mélange contenant 0,1 mole de $Si(OC_2H_5)_4$ et 0,00125 mole de $Ti(OC_4H_9)_4$ auquel on ajoute 2 g d'acétonylacétone, 2 g de n-butanol et 150 g d'eau. Le mélange, chauffé à reflux pendant 3 heures, se transforme en suspension colloïdale qui est ensuite évaporée et séchée à 80 ° C. On obtient ainsi 6,2 g d'un gel mixte ($SiO_2$, $TiO_2$) avec un rapport molaire Si/Ti = 80.

On disperse 1,85 g du gel mixte précédent avec 0,040 g de zéolite type MFI (germes) dans une solution aqueuse contenant 0,64 g de TPA-Br, 0,11 g de $NH_4F$ et 16,2 g d'eau. Le mélange réactionnel, qui a la composition molaire suivante, ramenée à 1 mole de $SiO_2$ :

1 $SiO_2$ ; 0,0125 $TiO_2$ ; 0,08 TPA-Br ; 0,1 $NH_4F$ ; 30 $H_2O$ est chauffé 4 jours à 200 ° C. Le solide cristallisé est séparé par filtration, lavé et séché puis calciné pendant 6 heures à 550 ° C.

L'analyse radiocristallographique montre qu'il s'agit d'une zéolite du type MFI pure avec un diagramme de diffraction RX conforme à celui du tableau I. L'analyse chimique donne un rapport molaire Si/Ti = 95.

Exemples 4

On réalise quatre mélanges réactionnels identiques selon le mode opératoire suivant :

On hydrolyse 2,72 g de $Ti(OC_4H_9)_4$ dans 20 ml d'eau, en agitant ce mélange pendant 6 heures. Le précipité obtenu est filtré puis dissout à chaud dans 20 ml d'eau en présence de 2,02 g d'acide oxalique ($C_2H_2O_4$). A cette solution d'oxalate de titane, on ajoute une solution contenant 5,33 g de bromure de tétrapropylammonium (TPA-Br), 1,48 g de fluorure d'ammonium ($NH_4F$) et 23,2 g d'eau. Après mélange, on disperse dans la solution 4,8 g de silice type Aerosil 130. La composition molaire ramenée à une mole de silice du mélange réactionnel est la suivante :

1 $SiO_2$ ; 0,1 $TiO_2$ ; 0,2 $C_2H_2O_4$ ; 0,25 TPA-Br ; 0,5 $NH_4F$ ; 30 $H_2O$.

Les quatre mélanges réactionnels sont ensuite cristallisés à 200 °C dans des autoclaves revêtus intérieurement par du PTFE suivant les procédures figurant dans le tableau II.

Tableau II - conditions opératoires

| Essai | Germes (1) | Agitation | Durée | Phases obtenues |
|---|---|---|---|---|
| 4a | 0 % | non | 2 jours | 35 % zéolite type MFI + 65 % amorphe et impuretés |
| 4b | 2 % | non | 1 jour | 75 % " " " + 25 % amorphe et impuretés |
| 4c | 2 % | non | 2 jours | > 90 % zéolite type MFI |
| 4d | 2 % | oui | 1 jour | > 90 % " " MFI |

(1) Les germes sont constitués d'une zéolite du type MFI. La quantité ajoutée est indiquée en % pondéral par rapport au poids de silice engagée.

Aprés cristallisation, les phases solides sont séparées par filtration, lavées à l'eau et séchées à 40 °C. Après calcination à 550 °C pendant 4 heures, les phases solides sont identifiées par leur spectre de diffraction des rayons X. On peut constater que la formation de zéolites du type MFI (titanozéosilite) est plus rapide en présence de germes et en milieu agité. L'analyse chimique effectuée sur le produit de l'exemple 4c donne un rapport molaire Si/Ti global de 25. L'estimation du rapport molaire Si/Ti dans la charpente de la zéolite peut être faite à partir de la mesure des déplacements relatifs des pics de diffraction. On trouve alors une valeur égale à environ 60.

Le spectre de diffraction des rayons X de la zéolite obtenue dans l'exemple 4c est indiqué dans le tableau IV.

Exemples 5

Les 7 essais de l'exemple 5 montrent que la cristallisation de la zéolite du type MFI (titanozésilite) ainsi que l'incorporation de l'élément Ti dans la charpente de la zéolite peut être contrôlé en jouant sur les quantités de mobilisateur ($F^-$) et de co-mobilisateur spécifique de Ti (acide oxalique). Ils ont été réalisés avec des mélanges réactionnels analogues à ceux utilisés dans l'exemple 4. Dans les essais 5b à 5f, l'utilisation de quantités d'acide oxalique plus faibles ainsi que l'absence d'acide oxalique dans l'essai 5g, ont pour conséquence une dissolution incomplète du précipité obtenu après hydrolyse de $Ti(OC_4H_9)_4$. Ils contiennent tous 2 % de germes de cristallisation. Les compositions molaires ramenées à une mole de silice ainsi que les pH figurent dans le tableau III.

Tableau III

| n° | $SiO_2$ | $TiO_2$ | TPA-Br | $NH_4F$ | $C_2H_2O_4$ | $H_2O$ | pH initial | pH final | Si/Ti estimé |
|---|---|---|---|---|---|---|---|---|---|
| 5a | 1 | 0,1 | 0,08 | 0,5 | 0,2 | 30 | 4 | 7 | 75 |
| 5b | 1 | 0,1 | 0,08 | 0,5 | 0,1 | 30 | 5 | 6 | 35 |
| 5c | 1 | 0,1 | 0,08 | 0,25 | 0,05 | 30 | 6 | 6 | 50 |
| 5d | 1 | 0,1 | 0,08 | 0,1 | 0,1 | 30 | 3 | 5 | 45 |
| 5e | 1 | 0,1 | 0,08 | 0,1 | 0,05 | 30 | 4 | 5,5 | 80 |
| 5f | 1 | 0,1 | 0,08 | 0 | 0,05 | 30 | 1,5 | 3,5 | amorphe |
| 5g | 1 | 0,1 | 0,08 | 0 | 0 | 30 | 6 | 6 | amorphe |

Les 7 mélanges réactionnels ont été chauffés 4 jours à 200 °C dans des autoclaves non agités et revêtus intérieurement de PTFE. Après filtration, lavage et séchage, les solides ont été calcinés 4 heures en creuset ouvert.

L'examen radiocristallographique des 7 échantillons montre qu'à l'exception des produits 5f et 5g, on a obtenu des zéolites du type MFI (titanozéosilite) bien cristallisées pouvant contenir quelques impuretés.

L'estimation du rapport Si/Ti (dernière colonne du tableau III) a été faite à partir de la mesure du déplacement relatif des pics de diffraction.

L'examen de ces résultats permet de tirer plusieurs conclusions. D'une part, la présence du mobilisateur ($F^-$) est nécessaire pour obtenir la cristallisation. D'autre part, le rapport Si/Ti peut être modifié en faisant varier les quantités de mobilisateur ($F^-$) et de co-mobilisateur $(COOH)_2$ mises en jeu. Des quantités trop importantes ou trop faibles de ces deux derniers constituants sont moins favorables à l'incorporation de l'élément Ti dans la charpente de la zéolite.

Exemple 6

L'exemple 6 illustre l'utilisation d'acétylacétone comme co-mobilisateur spécifique, à la place de l'acide oxalique.

On mélange 0,8 g d'acétylacétone avec 1,36 g de $Ti(OC_4H_9)_4$ et on ajoute sous forte agitation une solution contenant 0,37 g $NH_4F$ et 0,85 g TPA-Br dans 36 g $H_2O$ puis 2,40 g de silice aérosil et 0,040 g de germes (zéolite de type MFI). La composition molaire ramenée à 1 mole de silice est alors :

1 $SiO_2$ ; 0,1 $TiO_2$ ; 0,08 TPA-Br ; 0,25 $NH_4F$ ; 0,2 acétylacétone ; 50 $H_2O$.

La cristallisation du mélange réactionnel est effectuée dans les mêmes conditions que pour les essais de l'exemple 5. Après séparation et calcination, on obtient un solide cristallisé dont le diagramme de diffraction des rayons X est conforme à celui du tableau I. Le rapport Si/Ti dans la charpente est estimé par la mesure du déplacement relatif des pics de diffraction à une valeur voisine de 45.

Exemple 7 comparatif

A titre comparatif, une matière synthétique poreuse a été préparée selon le procédé décrit dans le brevet français n° 2 471 950 et appelée TS1 dans ce document.

Le procédé met en oeuvre une solution basique, utilisant comme agent mobilisateur des ions $OH^-$.

227,5 g de tétraéthylorthosilicate sont placés dans un récipient en verre pyrex muni d'un agitateur et maintenus sous une atmosphère exempte de $CO_2$ et 7,5 g de tétraéthyltitanate sont ajoutés, suivi d'une addition progressive de 500 g d'une solution à 20 % en poids d'hydroxyde de tétrapropylammonium.

Le mélange est maintenu agité pendant environ 1 heure avant un chauffage accélérant l'hydrolyse et l'évaporation de l'alcool éthylique.

Après 5 heures de chauffage à 80-90 °C, on ajoute de l'eau. La solution homogène opalescente est transférée dans un autoclave en inox muni d'un agitateur. Le mélange est chauffé à 165 °C et maintenu agité à cette température sous sa propre pression pendant une durée de 10 jours. L'autoclave est alors refroidi, et la masse de cristaux fins obtenus est récupérée. Après lavage des cristaux avec de l'eau, ceux-ci sont séchés puis calcinés pendant 6 heures à 550 °C.

Le spectre de diffraction des rayons X pour le produit calciné correspond à celui indiqué dans le tableau I du brevet FR 2 471 950.

Dans le tableau IV ci-dessous sont indiqués les spectres de diffraction des rayons X de la zéolite conforme à l'invention et obtenue dans l'exemple 4c et celui du produit de l'exemple comparatif 7.

Les différences de spectres entre ces deux produits sont évidentes et prouvent clairement que la zéolite obtenue avec comme agent mobilisateur des ions fluorures, contient des éléments titane dans sa charpente. Cette différence se concrétise par le système cristallin obtenu qui est monoclinique pour une zéolite de l'invention, et orthorhombique pour une silicalite de titane obtenue en milieu basique.

Tableau IV

Comparaison des diagrammes de diffraction RX correspondant à une titanozéosilite (exemple 4c) et à une silicalite de titane selon l'exemple comparatif 7

| Titanozéosilite (ex. 4c) | | Silicalite de titane (ex. 7) | | Titanozéosilite (ex. 4c) | | Silicalite de titane (ex. 7) | |
|---|---|---|---|---|---|---|---|
| $d_{hkl}$ (nm) | $I/I_c$ | $d_{hkl}$ (nm) | $I/I_c$ | $d_{hkl}$ (nm) | $I/I_c$ | $d_{hkl}$ (nm) | $I/I_c$ |
| 1,12 | F-FF | 1,114 | FF | 0,462 | f | | |
| 1,00 | F-FF | 0,999 | F | 0,446 | ff | | |
| 0,98 | f | 0,974 | m | 0,436 | f | 0,4360 | f |
| 0,90 | ff | | | 0,426 | f | 0,4260 | mf |
| 0,805 | ff | | | 0,4084 | ff | | |
| 0,746 | ff | | | 0,4008 | f | | |
| 0,709 | ff | | | 0,3859 | F | 0,3855 | F |
| 0,671 | F | 0,6702 | f | 0,3826 | mF | 0,3819 | F |
| 0,637 | f | 0,6362 | mf | 0,3806 | mF | | |
| 0,600 | fm | 0,5993 | mf | 0,3759 | m | 0,3751 | F |
| 0,594 | f | | | 0,3742 | m | | |
| 0,573 | f(m) | 0,5698 | f | 0,3718 | m | 0,3720 | F |
| 0,570 | épau. | | | 0,3663 | f | 0,3646 | m |
| 0,559 | f | 0,5574 | f | 0,3627 | f | | |
| 0,538 | f(f) | | | 0,3448 | f(f) | 0,3444 | f |
| 0,534 | f(f) | | | 0,3431 | f | | |
| 0,513 | ff | | | 0,3396 | ff | | |
| 0,504 | ff | 0,5025 | f | 0,3357 | f(f) | | |
| 0,498 | mf | 0,4980 | f | 0,3317 | f | 0,3318 | f |
| 0,488 | ff | | | 0,3256 | f | | |
| 0,471 | ff | | | | | | |

Exemple 8

La zéolite de l'invention peut être utilisée comme adsorbant, catalyseur et/ou support de catalyseur pour de nombreuses applications.

A titre d'exemple, on a testé son utilisation comme catalyseur de la réaction de déshydratation sélective du méthylbutanol en méthylbuténynе.

30 g de catalyseur préparé selon l'exemple 1 sont ajoutés dans 300 ml d'acide chlorhydrique 0,1N. Ce mélange est porté à 60 °C et maintenu à cette température sous agitation pendant 4 heures.

Le catalyseur est récupéré par filtration et lavé avec de l'eau. Après séchage en étuve à 100 °C, le catalyseur est broyé.

Ce catalyseur est disposé dans une colonne entre deux lits de billes de verre, et maintenu à 500 °C pendant une nuit pour activation.

Le méthylbutynol est alimenté en continu dans la colonne, sous atmosphère d'azote, à une température de 280 °C et sous un débit gazeux de 16 l/h.

Le produit récupéré est analysé par chromatographie en phase gazeuse.

Le pourcentage de méthylbuténynе dans le mélange de sortie est d'environ 85% pour un temps de réaction d'environ 2 heures.

Avec un catalyseur classique à base d'alumine gamma, ce taux est d'environ 65%.

Ces résultats montrent que le catalyseur de l'invention a une meilleure sélectivité.

Toutefois, le rendement de conversion du méthylbutanol est inférieur pour le catalyseur de l'invention par rapport au catalyseur à base d'alumine.

**Revendications**

**1.** Zéolite de structure MFI caractérisée en ce qu'elle est à base d'oxyde de silicium et d'oxyde de titane, et a la formule suivante, après calcination :

$Si_{(96-x)},Ti_x\ O_{192}$

dans laquelle : x est compris entre 0,1 et 6 environ et en ce qu'elle contient entre 0,01 et 0,8 % de fluor en poids, après calcination, et en ce qu'elle a une structure monoclinique et un diagramme de diffraction X défini dans le tableau I.

TABLEAU I

| diagramme de diffraction des rayons X | | | |
|---|---|---|---|
| Valeurs extrêmes des $d_{hkl}$ (nm) | $I/I_o$ | Valeurs extrêmes des $d_{hkl}$ (nm) | $I/I_o$ |
| 1,110-1,128 | F-FF | 0,3785-0,3845 | mF |
| 0,991-1,012 | F-FF | 0,3735-0,3795 | m |
| 0,972-0,986 | f | 0,3715-0,3775 | m |
| 0,895-0,906 | ff | 0,3705-0,3765 | m |
| 0,803-0,813 | ff | 0,3645-0,3700 | f |
| 0,741-0,753 | ff (large) | 0,3610-0,3670 | f |
| 0,704-0,715 | ff (large) | 0,3470-0,3525 | ff |
| 0,666-0,678 | f | 0,3430-0,3485 | f(f) |
| 0,632-0,643 | f | 0,3415-0,3470 | f(f) |
| 0,595-0,605 | mf | 0,3385-0,3439 | ff |
| 0,589-0,598 | f | 0,3341-0,3394 | f(f) |
| 0,568-0,577 | mf | 0,3290-0,3345 | f (large) |
| 0,565-0,574 | f (épaulement) | 0,3240-0,3292 | f |
| 0,555-0,564 | f | 0,3045-0,3099 | f(f) |
| 0,534-0,543 | f(f) | 0,3020-0,3068 | f |
| 0,531-0,539 | f(f) | 0,2978-0,3026 | f |
| 0,510-0,518 | ff | 0,2952-0,2999 | ff (épaulement) |
| 0,502-0,508 | ff | 0,2944-0,2991 | f |
| 0,496-0,504 | mf | 0,2914-0,2961 | ff |
| 0,485-0,493 | ff | 0,2852-0,2898 | ff (large) |
| 0,468-0,476 | ff | 0,2774-0,2818 | ff |
| 0,459-0,466 | f | 0,2722-0,2766 | ff (large) |
| 0,444-0,451 | f | 0,2676-0,2720 | ff |
| 0,433-0,441 | f | 0,2606-0,2648 | ff |
| 0,423-0,431 | f | 0,2586-0,2627 | ff |
| 0,4055-0,4125 | ff | 0,2544-0,2585 | ff (large) |
| 0,3985-0,4045 | f | 0,2508-0,2548 | ff |
| 0,3835-0,3905 | F | 0,2478-0,2518 | f |
| 0,3805-0,3865 | mF | | |

**2.** Zéolite de structure MFI à base d'oxyde de silicium et d'oxyde de titane, caractérisée par le fait qu'elle est obtenue selon un procédé comprenant les étapes suivantes :

(i) Préparation d'un mélange réactionnel en milieu aveux contenant au moins une source d'oxyde de silicium, une source d'oxyde de titane, des ions fluorures, un agent structurant, avec des rapports molaires Ti/Si compris entre 1,5 et 0,002, F/Si entre 10 et 0,04, $H_2O$/Si entre 400 et 4, agent structurant/Si entre 2 et 0,02 et et un pH compris entre 10,5 et 1,5.

(ii) Cristallisation du mélange réactionnel.

(iii) Récupération et calcination du précipité cristallin à une température supérieure à 450 °C.

**3.** Procédé de fabrication d'une zéolite selon l'une des revendications précédentes, caractérisé en ce qu'il comprend les étapes suivantes :

(i) Préparation d'un mélange réactionnel en milieu aqueux contenant au moins une source d'oxyde de silicium, une source d'oxyde de titane, des ions fluorures, un agent structurant, avec des rapports molaires Ti/Si compris entre 1,5 et 0,002, F/Si entre 10 et 0,04, $H_2O$/Si entre 400 et 4, agent structurant/Si entre 2 et 0,02 et un pH compris entre 10,5 et 1,5.

(ii) Cristallisation du mélange réactionnel.

(iii) Récupération et calcination du précipité cristallin à une température supérieure à 450 °C.

**4.** Procédé selon la revendication 3, caractérisé en ce que le rapport molaire Ti/Si dans le mélange réactionnel est compris entre 1 et 0,01.

**5.** Procédé selon la revendication 3 ou 4, caractérisé en ce que le rapport molaire F/Si dans le mélange réactionnel est compris entre 6 et 0,06.

**6.** Procédé selon l'une des revendications 3 à 5, caractérisé en ce que le rapport molaire $H_2O$/Si est compris entre 100 et 6.

**7.** Procédé selon l'une des revendications 3 à 6, caractérisé en ce que le rapport agent structurant/Si est compris entre 1 et 0,04.

**8.** Procédé selon l'une des revendications 3 à 7, caractérisé en ce que l'oxyde de silicium et l'oxyde de titane ont une source commune.

**9.** Procédé selon l'une des revendications 3 à 7, caractérisé en ce que l'agent structurant précité est choisi dans le groupe comprenant :

- les amines tertiaires de formule I :

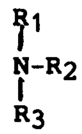

dans laquelle :
$R_1$, $R_2$, $R_3$ identiques ou différents représentent un groupe alkyle, de préférence un groupe propyle ou butyle.

- les ammonium quaternaires de formule II

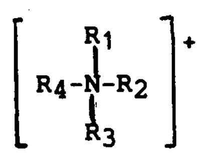

dans laquelle :
$R_1$, $R_2$, $R_3$, $R_4$ identiques ou différents représentent les groupes alkyles, de préférence les groupes propyles ou butyles.

- les composés de formule I ou II dans lesquelles l'azote a été remplacé par un atome de phosphore.

**10.** Procédé selon l'une des revendications 3 à 7 ou 9, caractérisé en ce que, la source d'oxyde de silicium est choisi dans le groupe comprenant les hydrogels, aérogels, suspensions colloïdales de silice, les esters siliciques, les silicates solubles dans l'eau, les silices extraites de composés cristallins naturels ou synthétiques, les composés du silicium tétravalent hydrolysables tels que les halogénures de silicium, et en ce que la source d'oxyde de titane est choisie dans le groupe comprenant les oxydes et hydroxydes de titane naturels ou synthétiques cristallisés ou amorphes, les halogénures, alcoolates, les sels titanates solubles dans l'eau.

**11.** Procédé selon l'une des revendications 3 à 10, caractérisé en ce que le mélange réactionnel précité comprend au moins un agent co-mobilisateur du titane dans un rapport molaire par rapport au silicium compris entre 3 et 0,01, de préférence entre 1 et 0,04.

**12.** Procédé selon la revendication 11, caractérisé en ce que l'agent co-mobilisateur est choisi dans le groupe comprenant l'acide oxalique et ses sels, l'acétylacétone, l'acide tartrique et ses sels.

**13.** Procédé selon l'une des revendications 3 à 12, caractérisé en ce que les ions fluorures sont ajoutés dans le mélange réactionnel sous la forme d' acide fluorhydrique, de fluorure d'ammonium ou d'amines, de composés hydrolysables libérant les anions fluorures.

14. Procédé selon la revendication 13, caractérisé en ce que les composés hydrolysables sont des sels fluorés contenant du silicium ou du titane, choisis dans le groupe comprenant les fluorures de titane, fluorure de silicium, fluorure double d'ammonium et de titane, fluorure double d'ammonium et de silicium.

15. Composé cristallin contenant de la silice et de l'oxyde de titane caractérisé en ce qu'il est susceptible d'être obtenu par le procédé comprenant les étapes suivantes :
    (i) Préparation d'un mélange réactionnel en milieu aqueux contenant au moins une source d'oxyde de silicium, une source d'oxyde de titane, des ions fluorures, un agent structurant, avec des rapports molaires Ti/Si compris entre 1,5 et 0,002, F/Si entre 10 et 0,04, $H_2O$/Si entre 400 et 4 et structurant/Si entre 2 et 0,02 et un pH compris entre 10,5 et 5.
    (ii) Cristallisation du mélange réactionnel.
    (iii) Récupération du précipité cristallin.

16. Catalyseur pour la déshydratation du méthylbutanol en méthylbutanyne caractérisé en ce qu'il contient une zéolite selon l'une des revendications 1 ou 2.

## Claims

1. Zeolite of MFI structure, characterised in that it is based on silicon oxide and titanium oxide and has the following formula after calcination:

$$Si_{(96-x)}Ti_x \cdot O_{192}$$

in which: x is approximately between 0.1 and 6 and in that it contains between 0.01 and 0.8 % by weight of fluorine, after calcination, and in that it has a monoclinic structure and an x-ray diffraction pattern defined in Table I.

TABLE I

| X-ray diffraction pattern | | | |
|---|---|---|---|
| Outermost values of $d_{hkl}$ (nm) | $I/I_o$ | Outermost values of $d_{hkl}$ (nm) | $I/I_o$ |
| 1.110-1.128 | S-VS | 0.3785-0.3845 | mS |
| 0.991-1.012 | S-VS | 0.3735-0.3795 | m |
| 0.972-0.986 | w | 0.3715-0.3775 | m |
| 0.895-0.906 | vw | 0.3705-0.3765 | m |
| 0.803-0.813 | vw | 0.3645-0.3700 | w |
| 0.741-0.753 | vw (broad) | 0.3610-0.3670 | w |
| 0.704-0.715 | vw (broad) | 0.3470-0.3525 | vw |
| 0.666-0.678 | w | 0.3430-0.3485 | w(w) |
| 0.632-0.643 | w | 0.3415-0.3470 | w(w) |
| 0.595-0.605 | mw | 0.3385-0.3439 | vw |
| 0.589-0.598 | w | 0.3341-0.3394 | w(w) |
| 0.568-0.577 | mw | 0.3290-0.3345 | w (broad) |
| 0.565-0.574 | w (shoulder) | 0.3240-0.3292 | w |
| 0.555-0.564 | w | 0.3045-0.3099 | w(w) |
| 0.534-0.543 | w(w) | 0.3020-0.3068 | w |
| 0.531-0.539 | w(w) | 0.2978-0.3026 | w |
| 0.510-0.518 | vw | 0.2952-0.2999 | vw (shoulder) |
| 0.502-0.508 | vw | 0.2944-0.2991 | w |
| 0.496-0.504 | mw | 0.2914-0.2961 | vw |
| 0.485-0.493 | vw | 0.2852-0.2898 | vw (broad) |
| 0.468-0.476 | vw | 0.2774-0.2818 | vw |
| 0.459-0.466 | w | 0.2722-0.2766 | vw (broad) |
| 0.444-0.451 | w | 0.2676-0.2720 | vw |
| 0.433-0.441 | w | 0.2606-0.2648 | vw |
| 0.423-0.431 | w | 0.2586-0.2627 | vw |
| 0.4055-0.4125 | vw | 0.2544-0.2585 | vw (broad) |
| 0.3985-0.4045 | w | 0.2508-0.2548 | vw |
| 0.3835-0.3905 | S | 0.2478-0.2518 | w |
| 0.3805-0.3865 | mS | | |

**2.** Zeolite of MFI structure based on silicon oxide and titanium oxide, characterised by the fact that it is obtained according to a process comprising the following stages:

i) Preparation of a reaction mixture in an aqueous medium containing at least one source of silicon oxide, a source of titanium oxide, fluoride ions, a structuring agent, with molar ratios of Ti/Si of between 1.5 and 0.002, F/Si of between 10 and 0.04, $H_2O$/Si of between 400 and 4, structuring agent/Si of between 2 and 0.02 and a pH of between 10.5 and 1.5.

(ii) Crystallisation of the reaction mixture.

(iii) Recovery and calcination of the crystalline precipitate at a temperature above 450 °C.

**3.** Process for the manufacture of a zeolite according to either of the preceding claims, characterised in that it comprises the following stages:

i) Preparation of a reaction mixture in an aqueous medium containing at least one source of silicon oxide, a source of titanium oxide, fluoride ions, a structuring agent, with molar ratios of Ti/Si of between 1.5 and 0.002, F/Si of between 10 and 0.04, $H_2O$/Si of between 400 and 4, structuring agent/Si of between 2 and 0.02 and a pH of between 10.5 and 1.5.

(ii) Crystallisation of the reaction mixture.

(iii) Recovery and calcination of the crystalline precipitate at a temperature above 450 °C.

**4.** Process according to Claim 3, characterised in that the molar ratio Ti/Si in the reaction mixture is between 1 and 0.01.

**5.** Process according to Claim 3 or 4, characterised in that the molar ratio F/Si in the reaction mixture is between 6 and 0.06.

**6.** Process according to one of Claims 3 to 5, characterised in that the molar ratio $H_2O$/Si is between 100 and 6.

**7.** Process according to one of Claims 3 to 6, characterised in that the ratio structuring agent/Si is between 1 and 0.04.

**8.** Process according to one of Claims 3 to 7, characterised in that the silicon oxide and the titanium oxide have a common source.

**9.** Process according to one of Claims 3 to 7, characterised in that the abovementioned structuring agent is chosen from the group comprising:

- the tertiary amines of formula I:

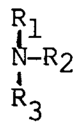

in which:
$R_1$, $R_2$, and $R_3$, which are identical or different, denote an alkyl group, preferably a propyl or butyl group,

- the quaternary ammoniums of formula II

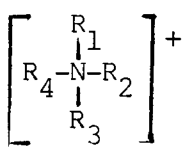

in which:
$R_1$, $R_2$, $R_3$ and $R_4$, which are identical or different, denote alkyl groups, preferably propyl or butyl groups, and

- the compounds of formula I or II in which the nitrogen has been replaced by a phosphorus atom.

**10.** Process according to one of Claims 3 to 7 or 9, characterised in that the source of silicon oxide is chosen from the group comprising hydrogels, aerogels and colloidal suspensions of silica, silicic esters, water-soluble silicates, silicas extracted from natural or synthetic crystalline compounds, hydrolysable tetravalent silicon compounds such as silicon halides, and in that the source of titanium oxide is chosen from the group comprising crystalline or amorphous natural or synthetic titanium oxides and hydroxides, halides, alcoholates and water-soluble titanate salts.

**11.** Process according to one of Claims 3 to 10, characterised in that the abovementioned reaction mixture comprises at least one comobilising agent for titanium in a molar ratio relative to the silicon of between 3 and 0.01, preferably between 1 and 0.04.

**12.** Process according to Claim 11, characterised in that the comobilising agent is chosen from the group comprising oxalic acid and its salts, acetylacetone, and tartaric acid and its salts.

**13.** Process according to one of Claims 3 to 12, characterised in that the fluoride ions are added to the reaction mixture in the form of hydrofluoric acid, ammonium or amine fluoride, or of hydrolysable compounds which release fluoride anions.

**14.** Process according to Claim 13, characterised in that the hydrolysable compounds are fluorine-containing salts containing silicon or titanium, chosen from the group comprising titanium fluorides, silicon fluoride, ammonium titanium fluoride, and ammonium silicon fluoride.

**15.** Crystalline compound containing silica and titanium oxide, characterised in that it is capable of being obtained by the process comprising the following stages:
(i) Preparation of a reaction mixture in an aqueous medium containing at least one source of silicon oxide, a source of titanium oxide, fluoride ions, a structuring agent, with molar ratios of Ti/Si of between 1.5 and 0.002, F/Si of between 10 and 0.04, $H_2O$/Si of between 400 and 4 and structuring agent/Si of between 2 and 0.02 and a pH of between 10.5 and 5.
(ii) Crystallisation of the reaction mixture.
(iii) Recovery of the crystalline precipitate.

**16.** Catalyst for the dehydration of methylbutanol to methylbutanyne, characterised in that it contains a zeolite according to either of Claims 1 and 2.

**Patentansprüche**

**1.** Zeolith mit MFI-Struktur
dadurch **gekennzeichnet,** daß
er auf der Basis von Siliciumoxid und Titanoxid nach dem Brennen der folgenden Formel

$Si_{(96-x)},Ti_x\ O_{192}$

entspricht, in der x im Bereich von etwa 0,1 bis 6 liegt und daß er nach dem Brennen 0,01 bis 0,8 Gewichts% Fluor enthält und daß er eine monokline Struktur und ein Röntgenbeugungsdiagramm, wie in Tabelle 1 definiert, aufweist.

Tabelle 1

| Röntgenbeugungsdiagramm | | | |
|---|---|---|---|
| Extremwerte der $d_{hkl}$ (nm) | $I/I_o$ | Extremwerte der $d_{hkl}$ (nm) | $I/I_o$ |
| 1,110-1,128 | F-FF | 0,3785-0,3845 | mF |
| 0,991-1,012 | F-FF | 0,3735-0,3795 | m |
| 0,972-0,986 | f | 0,3715-0,3775 | m |
| 0,895-0,906 | ff | 0,3705-0,3765 | m |
| 0,803-0,813 | ff | 0,3645-0,3700 | f |
| 0,741-0,753 | ff (breit) | 0,3610-0,3670 | f |
| 0,704-0,715 | ff (breit) | 0,3470-0,3525 | ff |
| 0,666-0,678 | f | 0,3430-0,3485 | f(f) |
| 0,632-0,643 | f | 0,3415-0,3470 | f(f) |
| 0,595-0,605 | mf | 0,3385-0,3439 | ff |
| 0,589-0,598 | f | 0,3341-0,3394 | f(f) |
| 0,568-0,577 | mf | 0,3290-0,3345 | f (breit) |
| 0,565-0,574 | f (Schulter) | 0,3240-0,3292 | f |
| 0,555-0,564 | f | 0,3045-0,3099 | f(f) |
| 0,534-0,543 | f(f) | 0,3020-0,3068 | f |
| 0,531-0,539 | f(f) | 0,2978-0,3026 | f |
| 0,510-0,518 | ff | 0,2952-0,2999 | ff (Schulter) |
| 0,502-0,508 | ff | 0,2944-0,2991 | f |
| 0,496-0,504 | mf | 0,2914-0,2961 | ff |
| 0,485-0,493 | ff | 0,2852-0,2898 | ff (breit) |
| 0,468-0,476 | ff | 0,2774-0,2818 | ff |
| 0,459-0,466 | f | 0,2722-0,2766 | ff (breit) |
| 0,444-0,451 | f | 0,2676-0,2720 | ff |
| 0,433-0,441 | f | 0,2606-0,2648 | ff |
| 0,423-0,431 | f | 0,2586-0,2627 | ff |
| 0,4055-0,4125 | ff | 0,2544-0,2585 | ff (breit) |
| 0,3985-0,4045 | f | 0,2508-0,2548 | ff |
| 0,3835-0,3905 | F | 0,2478-0,2518 | f |
| 0,3805-0,3865 | mF | | |
| F = stark<br>FF = sehr stark<br>m = mäßig<br>mf = mäßig bis schwach<br>mF = mäßig bis stark<br>f = schwach<br>ff = sehr schwach | | | |

**2.** Zeolith mit MFI-Struktur auf der Basis von Siliciumoxid und Titanoxid dadurch **gekennzeichnet,** daß er mit Hilfe eines Verfahrens erhalten wird, das folgende Stufen umfaßt:

(i) Herstellung eines Reaktionsgemisches in wäßrigem Medium, das mindestens eine Siliciumoxidquelle, eine Titanoxidquelle, Fluoridionen und ein strukturgebendes Mittel enthält, wobei die Molverhältnisse Ti/Si im Bereich von 1,5 bis 0,002, F/Si im Bereich 10 bis 0,04, $H_2O$/Si im Bereich von 400 bis 4 und strukturgebendes Mittel/Si im Bereich von 2 bis 0,02 liegen und der pH-Wert im Bereich von 10,5 bis 1,5 liegt,

(ii) Kristallisation des Reaktionsgemisches,

(iii) Isolieren und Brennen des kristallinen Niederschlags bei einer Temperatur oberhalb 450 °C

**3.** Verfahren zur Herstellung eines Zeoliths nach einem der vorangehenden Ansprüche,
dadurch **gekennzeichnet,** daß
es folgende Stufen umfaßt:

(i) Herstellung eines Reaktionsgemisches in wäßrigem Medium, das mindestens eine Siliciumoxidquelle, eine Titanoxidquelle, Fluoridionen und ein strukturgebendes Mittel enthält, wobei die Molverhältnisse Ti/Si im Bereich von 1,5 bis 0,002, F/Si im Bereich 10 bis 0,04, $H_2O$/Si im Bereich von 400 bis 4 und strukturgebendes Mittel/Si im Bereich von 2 bis 0,02 liegen und der pH-Wert im Bereich von 10,5 bis 1,5 liegt,
(ii) Kristallisation des Reaktionsgemisches,
(iii) Isolieren und Brennen des kristallinen Niederschlags bei einer Temperatur oberhalb 450 °C

**4.** Verfahren nach Anspruch 3,
dadurch **gekennzeichnet,** daß
das Molverhältnis Ti/Si im Reaktionsgemisch im Bereich von 1 bis 0,01 liegt.

**5.** Verfahren nach Anspruch 3 oder 4,
dadurch **gekennzeichnet,** daß
das Molverhältnis F/Si im Rekationsgemisch im Bereich von 6 bis 0,06 liegt.

**6.** Verfahren nach einem der Ansprüche 3 bis 5,
dadurch **gekennzeichnet,** daß
das Molverhältnis $H_2O$/Si im Bereich von 100 bis 6 liegt.

**7.** Verfahren nach einem der Ansprüchen 3 bis 6,
dadurch **gekennzeichnet,** daß
das Verhältnis von strukturgebendem Mittel/Si im Bereich von 1 bis 0,04 liegt.

**8.** Verfahren nach einem der Ansprüche 3 bis 7,
dadurch **gekennzeichnet,** daß
das Siliciumoxid und Titanoxid eine gemeinsame Quelle haben.

**9.** Verfahren nach einem der Ansprüche 3 bis 7,
dadurch **gekennzeichnet,** daß
das genannte strukturgebende Mittel aus der Gruppe ausgewählt wird, die umfaßt:

- tertiäre Amine der Formel I:

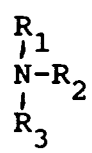

in der $R_1$, $R_2$ und $R_3$ gleich oder verschieden sind und eine Alkylgruppe, vorzugsweise eine Propyl- oder Butylgruppe bedeuten.

- quaternäre Ammoniumverbindungen der Formel II

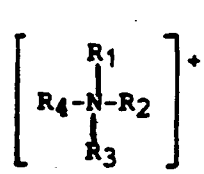

in der $R_1$, $R_2$, $R_3$ und $R_4$ gleich oder verschieden sind und Alkylgruppen, vorzugsweise Propyl- oder Butylgruppen bedeuten.

- Verbindungen der Formel I oder II, bei denen das Stickstoffatom durch ein Phosphoratom ersetzt worden ist.

**10.** Verfahren nach einem der Ansprüche 3 bis 7 oder 9,
dadurch **gekennzeichnet,** daß
die Siliciumoxidquelle ausgewählt wird aus der Gruppe der Hydrogele, Aerogele und kolloidalen

Suspensionen von Kieselsäure, den Kieselsäureestern, wasserlöslichen Silicaten, aus natürlichen oder synthetischen kristallinen Verbindungen extrahierte Kieselsäure und hydrolysierbaren Verbindungen von 4-wertigem Silicium, wie Siliciumhalogenide und daß die Titanoxidquelle ausgewählt wird aus der Gruppe der natürlichen oder synthetischen, kristallisierten oder amorphen Oxide und Hydroxide von Titan, den Halogeniden, Alkoholaten und den wasserlöslichen Titanatsalzen.

**11.** Verfahren nach einem der Ansprüche 3 bis 10,
dadurch **gekennzeichnet,** daß
das genannte Reaktionsgemisch mindestens ein als Co-Mobilisator von Titan wirkendes Mittel in einem Molverhältnis bezogen auf Silicium von 3 bis 0,01 vorzugsweise von 1 bis 0,04 enthält.

**12.** Verfahren nach Anspruch 11,
dadurch **gekennzeichnet,** daß
das als Co-Mobilisator wirkende Mittel aus der Gruppe ausgewählt wird, die Oxalsäure und ihre Salze, Acetylaceton, sowie Weinsäure und ihre Salze, umfaßt.

**13.** Verfahren nach einem der Ansprüche 3 bis 12,
dadurch **gekennzeichnet,** daß
die Fluoridionen dem Reaktionsgemisch in Form von Fluorwasserstoffsäure, Ammoniumfluorid oder Aminfluoriden oder von hydrolysierbaren Verbindungen, die Fluoridanionen freisetzen, zugegeben werden.

**14.** Verfahren nach Anspruch 13,
dadurch **gekennzeichnet,** daß
die hydrolysierbaren Verbindungen fluorhaltige Salze sind, die Silicium oder Titan enthalten und die aus der Gruppe umfassend die Fluoride von Titan, Siliciumfluorid, Ammonium-Titandoppelfluorid, Ammonium-Siliciumdoppelfluorid ausgewählt sind.

**15.** Kristalline Verbindung, die Siliciumoxid und Titanoxid enthält, dadurch **gekennzeichnet,** daß
sie mit Hilfe des Verfahrens, das folgende Stufen umfaßt erhalten werden kann:
(i) Herstellung eines Reaktionsgemisches in wäßrigem Medium, das mindestens eine Siliciumoxidquelle, eine Titanoxidquelle, Fluoridionen und ein strukturgebendes Mittel enthält, wobei die Molverhältnisse Ti/Si im Bereich von 1,5 bis 0,002, F/Si im Bereich 10 bis 0,04, $H_2O$/Si im Bereich von 400 bis 4 und strukturgebendes Mittel/Si im Bereich von 2 bis 0,02 liegen und der pH-Wert im Bereich von 10,5 bis 1,5 liegt,
(ii) Kristallisation des Reaktionsgemisches,
(iii) Isolieren und Brennen des kristallinen Niederschlags bei einer Temperatur oberhalb 450 ° C

**16.** Katalysator für die Dehydratisierung von Methylbutanol zu Methylbutanin,
dadurch **gekennzeichnet,** daß
er einen Zeolithen nach einem der Ansprüche 1 oder 2 enthält.